# EUROPEAN PATENT APPLICATION

(11) **EP 1 679 311 A1**
(43) Date of publication of application: **12.07.2006**
(21) Application number: 04793373.4
(22) Date of filing: 29.10.2004
(51) Int. Cl.: C07D 317/42, C07D 317/26, C08F 24/00, G02B 6/00, G02B 6/12

(54) **NOVEL FLUORINE COMPOUND AND FLUOROPOLYMER**

(30) Priority: 31.10.2003 JP 2003372653
(71) Applicant: ASAHI GLASS COMPANY LTD., Tokyo 100-8405 (JP)
(72) Inventor: ERIGUCHI, Takeshi c/o Asahi Glass Company, Limited, Yokohama-shi, Kanagawa 2218755 (JP); OKAZOE, Takashi, c/o Asahi Glass Company, Limited, Yokohama-shi, Kanagawa 2218755 (JP); MUROTANI, Eisuke, c/o Asahi Glass Company, Limited, Yokohama-shi, Kanagawa 2218755 (JP); ITO, Masahiro, c/o Asahi Glass Company, Limited, Yokohama-shi, Kanagawa 2218755 (JP); WATANABE, Kunio, c/o Asahi Glass Company, Limited, Yokohama-shi, Kanagawa 2218755 (JP)
(74) Representative: Müller-Boré & Partner Patentanwälte
(86) International application number: PCT/JP2004/016453
(87) International publication number: WO 2005/042511

(57) **Abstract**

The present invention provides a fluoropolymer having excellent mechanical strength and transparency, having a high glass transition point suitable for high-temperature use, and having a high refractive index.

The present invention provides a novel compound represented by the following formula (1) and a fluoropolymer comprising monomer unit (2) formed by the polymerization of such a compound. R^{AF}, R^{BF}, R^{CF}, R^{DF}: each independently is a fluorine atom, a chlorine atom, a perfluoro monovalent saturated hydrocarbon group or a perfluoro(partially chlorinated monovalent saturated hydrocarbon) group, provided that at least one of R^{AF}, R^{BF}, R^{CF} and R^{DF} is a perfluoro(partially chlorinated monovalent saturated hydrocarbon) group.

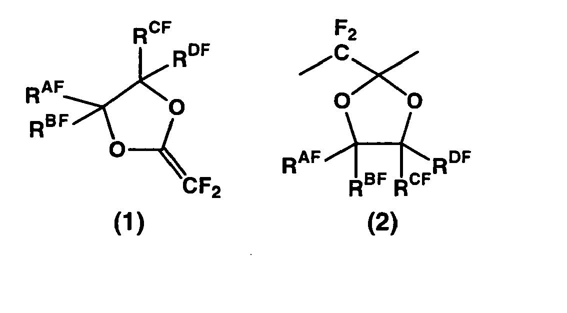

## Description

### TECHNICAL FIELD

The present invention relates to a novel fluorinated compound having a perfluoro(2-methylene-1,3-dioxolane) structure and having a perfluoro(partially chlorinated monovalent saturated hydrocarbon) group at the 4-position and/or 5-position of the structure, and a fluoropolymer obtained by polymerizing such a fluorinated compound.

### BACKGROUND ART

A fluoropolymer having saturated cyclic structures in its main chain, is useful as an optical material because of the non-crystallinity and the excellent mechanical strength and transparency. In recent years, such a fluoropolymer is desired to have a high glass transition point suitable for use at a high temperature and an optional refractive index depending upon the particular application.

The fluoropolymer having saturated cyclic structures in its main chain, may be a fluoropolymer wherein carbon-carbon bonds in its main chain are contained in the saturated cyclic structures, and a fluoropolymer other than such a fluoropolymer. As the latter fluoropolymer, a polymer containing monomer units represented by the following formula (D) is known, and it is disclosed in JP-A-5-213929 and JP-A-5-339255 that such a polymer can be obtained by polymerizing a compound represented by the following formula (d): 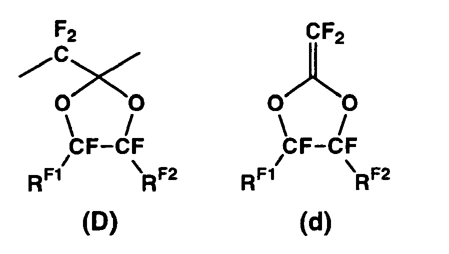 wherein R^{F1} is a fluorine atom or -CF₃, and when R^{F1} is a fluorine atom, R^{F2} is a fluorine atom, -CF₃ or -(CF₂)₃F and when R^{F1} is -CF₃, R^{F2} is -CF₃.

Further, Example 17 (page 11) in JP-A-2-124908 discloses a compound represented by the following formula (e), and a polymer made of monomer units represented by the following formula (E) obtained by homopolymerizing such a compound.

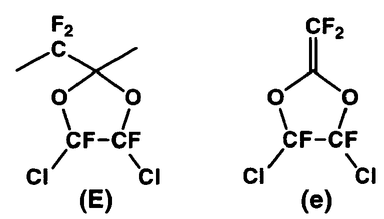

The polymer containing monomer units represented by the formula (D) has high transparency and excellent thermal stability, but it is insufficient in the refractive index, and its application as an optical material was limited. For example, in a case where such a polymer was used for a core of an optical waveguide, it was found difficult to make a large difference in refractive index from a clad. Further, the polymer containing monomer units represented by the formula (E) was insufficient in the mechanical strength and thermal stability.

### DISCLOSURE OF THE INVENTION

The present invention has been made to solve the above problems, and it is an object of the present invention to provide a novel fluoropolymer having a high refractive index which is used as an optical material such as an optical waveguide material. Further, it is an object of the present invention to provide a fluoropolymer having mechanical strength, transparency, and a high glass transition point, as physical properties required for the optical material.

The present invention provides a compound represented by the following formula (1).
<1> A compound represented by the following formula (1) : 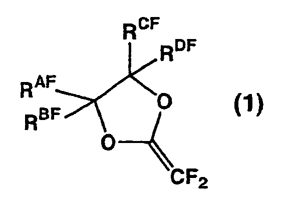
   R^{AF}, R^{BF}, R^{CF}, R^{DF}: each independently is a fluorine atom, a chlorine atom, a perfluoromonovalent saturated hydrocarbon group or a perfluoro(partially chlorinated monovalent saturated hydrocarbon) group, provided that at least one of R^{AF}, R^{BF}, R^{CF} and R^{DF} is a perfluoro(partially chlorinated monovalent saturated hydrocarbon) group.
<2> The compound according to <1>, wherein each of R^{AF} and R^{CF} which are independent of each other, is a perfluoro(partially chlorinated monovalent saturated hydrocarbon) group, and R^{BF} and R^{DF} are fluorine atoms, or R^{AF} is a perfluoro(partially chlorinated monovalent saturated hydrocarbon) group, and R^{BF}, R^{CF} and R^{DF} are fluorine atoms.
<3> The compound according to <1> or <2>, wherein the perfluoro(partially chlorinated monovalent saturated hydrocarbon) group is a C₁₋₆ perfluoro(partially chlorinated alkyl) group.
<4> The compound according to any one of <1> to <3>, wherein the ratio of the number of chlorine atoms in the compound represented by the formula (1) to the number of carbon atoms in the same compound, is from 0.1 to 0.5.
<5> A fluoropolymer comprising monomer units represented by the following formula (2): 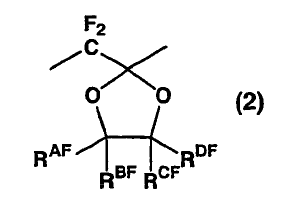
   R^{AF}, R^{BF}, R^{CF}, R^{DF}: each independently is a fluorine atom, a chlorine atom, a perfluoromonovalent saturated hydrocarbon group or a perfluoro(partially chlorinated monovalent saturated hydrocarbon) group, provided that at least one of R^{AF}, R^{BF}, R^{CF} and R^{DF} is a perfluoro(partially chlorinated monovalent saturated hydrocarbon) group.
<6> The fluoropolymer according to <5>, wherein each of R^{AF} and R^{CF} which are independent of each other, is a perfluoro(partially chlorinated monovalent saturated hydrocarbon) group, and R^{BF} and R^{DF} are fluorine atoms, or R^{AF} is a perfluoro(partially chlorinated monovalent saturated hydrocarbon) group, and R^{BF}, R^{CF} and R^{DF} are fluorine atoms.
<7> The fluoropolymer according to <5> or <6>, wherein the perfluoro(partially chlorinated monovalent saturated hydrocarbon) group is a C₁₋₆ perfluoro(partially chlorinated alkyl) group.
<8> The fluoropolymer according to any one of <5> to <7>, wherein the ratio of the number of chlorine atoms in the monomer units represented by the formula (2) to the number of carbon atoms in the same monomer units, is from 0.1 to 0.5.
<9> The fluoropolymer according to any one of <5> to <8>, which comprises at least one type of monomer units represented by the formula (2).
<10> The fluoropolymer according to any one of <5> to <9>, which has a number average molecular weight of from 5,000 to 5,000,000.
<11> A process for producing a fluoropolymer comprising monomer units represented by the following formula (2), characterized by polymerizing a compound represented by the following formula (1), or copolymerizing a compound represented by the following formula (1) with another monomer polymerizable with the compound: 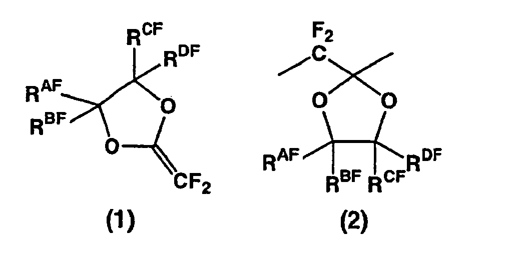
   R^{AF}, R^{BF}, R^{CF}, R^{DF}: each independently is a fluorine atom, a chlorine atom, a perfluoromonovalent saturated hydrocarbon group or a perfluoro(partially chlorinated monovalent saturated hydrocarbon) group, provided that at least one of R^{AF}, R^{BF}, R^{CF} and R^{DF} is a perfluoro(partially chlorinated monovalent saturated hydrocarbon) group.
<12> An optical material comprising as an effective component the fluoropolymer as defined in any one of <5> to <10>.

### BEST MODE FOR CARRYING OUT THE INVENTION

In the present specification, the compound represented by the formula (1) will be referred to as the compound (1). Compounds represented by other formulae will be likewise referred to. Further, monomer units represented by formula (2) will be referred to as monomer units (2). Here, monomer units are meant for structural units derived from a monomer, which are formed by polymerization of the monomer.

In the present specification, a group having a structure in which one or more hydrogen atoms bonded to carbon atoms are substituted by fluorine atoms, is represented by adding "polyfluoro" before the name of the group. In such a polyfluoro group, hydrogen atoms may or may not be present. A group having a structure in which substantially all hydrogen atoms bonded to carbon atoms are substituted by fluorine atoms, is represented by adding "perfluoro" before the name of the group. In such a perfluoro group, no hydrogen atom is present. A group in which some of hydrogen atoms bonded to carbon atoms are substituted by chlorine atoms, is represented by adding "partially chlorinated" before the name of the group. In such a partially chlorinated group, hydrogen atoms are present.

In the partially chlorinated group in which some of hydrogen atoms bonded to carbon atoms are substituted by chlorine atoms, a group in which substantially all the remaining hydrogen atoms are substituted by fluorine atoms, is represented by adding "perfluoro" before the name of the group. In such a perfluoro partially chlorinated group, no hydrogen atom is present.

The number of carbon atoms of the monovalent hydrocarbon group in the present specification is preferably from 1 to 20, more preferably from 1 to 10, particularly preferably from 1 to 6. The structure of the group may, for example, be a linear structure, a branched structure, a cyclic structure or a structure partially having a cyclic structure. A carbon-carbon bond in the group may be a single bond, a double bond or a triple bond.

The carbon number of the monovalent saturated hydrocarbon group, the perfluoro monovalent saturated hydrocarbon group or the perfluoro(partially chlorinated monovalent saturated hydrocarbon) group in the present specification, is preferably from 1 to 20, more preferably from 1 to 10, particularly preferably from 1 to 6, and more particularly preferably from 1 to 4. Further, the structure of the group may, for example, be a linear structure, a branched structure, a cyclic structure or a structure partially having a cyclic structure.

The compound (1) in the present invention is represented by the following formula (1):

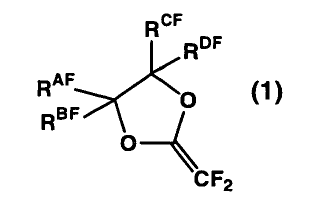

R^{AF}, R^{BF}, R^{CF}, R^{DF}: each independently is a fluorine atom, a chlorine atom, a perfluoro monovalent saturated hydrocarbon group or a perfluoro(partially chlorinated monovalent saturated hydrocarbon) group, provided that at least one of R^{AF}, R^{BF}, R^{CF} and R^{DF} is a perfluoro(partially chlorinated monovalent saturated hydrocarbon) group.

The compound (1) of the present invention is a compound characterized by having a perfluoro(2-methylene-1,3-dioxolane) structure and having a structure in which a perfluoro(partially chlorinated monovalent saturated hydrocarbon) group is bonded at the 4-position and/or 5-position of the structure. It is considered that the perfluoro(partially chlorinated monovalent saturated hydrocarbon) group in the structure contributes to the refractive index and thermal stability of the after-mentioned fluoropolymer (2).

Further, the compound (1) of the present invention is characterized by essentially having a group containing chlorine atoms. The number of the groups containing chlorine atoms is preferably determined by the number of total carbon atoms in the compound (1) and the number of chlorine atoms in the group. The number of carbon atoms in the compound (1) is preferably from 4 to 24, more preferably from 4 to 10, particularly preferably from 4 to 8.

The number of chlorine atoms in a perfluoro(partially chlorinated monovalent saturated hydrocarbon) group is preferably 1 or 2. The perfluoro(partially chlorinated monovalent saturated hydrocarbon) group is preferably a perfluoro(partially chlorinated alkyl) group, more preferably such a group having from 1 to 6 carbon atoms, particularly preferably such a group having from 1 to 4 carbon atoms. As a specific example of such a group, -CF₃₋ₚClₚ, -C₂F_{5-q}Cl_{q}, -C₃F₇₋ᵣClᵣ or -C₄F₉₋ₛClₛ (provided that a group having at least 3 carbon atoms may be a linear structure or a branched structure) may be mentioned, wherein p is an integer of 1 or 2, q is an integer of from 1 to 4, r is an integer of from 1 to 6 and s is an integer of from 1 to 8. p is preferably 1, and each of q, r and s which are independent of one another, is preferably 1 or 2.

The ratio of the number of chlorine atoms to the number of carbon atoms in the compound (1) is preferably more than 0 and less than 1.5, more preferably from 0.1 to 0.5 from the viewpoint of the chemical stability and transparency of the after-mentioned fluoropolymer (2), particularly preferably from 0.1 to 0.4 if the mechanical strength of the after-mentioned fluoropolymer (2) is taken into consideration. The number of chlorine atoms in the structure contributes to the refractive index of the after-mentioned fluoropolymer (2).

In a case where R^{AF} to R^{DF} are perfluoromonovalent saturated hydrocarbon groups, they are preferably perfluoroalkyl groups, particularly preferably C₁₋₆ perfluoroalkyl groups, most preferably -CF₃, -C₂F₅ or -(CF₂)₃F.

R^{AF} to R^{DF} are preferably such that they are fluorine atoms or perfluoro(partially chlorinated monovalent saturated hydrocarbon) groups and at least one of them is a perfluoro(partially chlorinated monovalent saturated hydrocarbon) group, particularly preferably such that R^{AF} is a perfluoro(partially chlorinated monovalent saturated hydrocarbon) group and R^{BF}, R^{CF} and R^{DF} are fluorine atoms, or R^{AF} and R^{CF} are perfluoro(partially chlorinated monovalent saturated hydrocarbon) groups and R^{BF} and R^{DF} are fluorine atoms.

As specific examples of the compound (1), the following compounds may be mentioned (wherein s is as defined above).

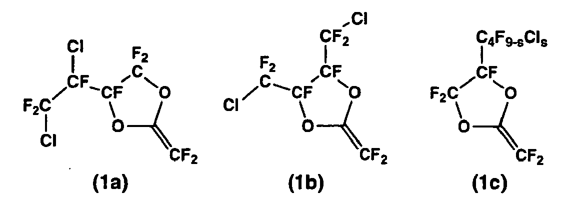

The compound (1) can be produced by a production process represented by the following formulae, wherein the following compound (5) is perfluorinated by a fluorination reaction (preferably a liquid phase fluorination reaction) to obtain the following compound (4), then an ester linkage of the compound (4) is decomposed to obtain the following compound (3), and then the compound (3) is thermally decomposed. The reaction condition, reaction operation, etc. in the production process are preferably in accordance with the process as described in WO01/16085, WO02/10106 or WO03/037885 by the present applicants. Further, the compound (3) may be converted to an alkali metal salt represented by the following compound (3A), followed by thermal decomposition: 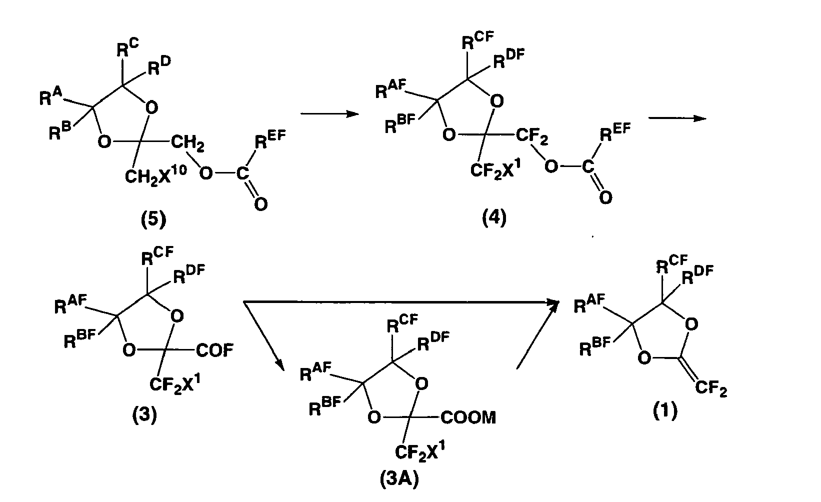 In the formulae, R^{AF} to R^{DF} are as defined above. X¹ is a fluorine atom or a chlorine atom, preferably a fluorine atom. X¹⁰ corresponds to X¹, and when X¹ is a chlorine atom, X¹⁰ is a chlorine atom, and when X¹ is a fluorine atom, X¹⁰ is a hydrogen atom or a fluorine atom, preferably a hydrogen atom. M is an alkali metal atom, preferably a potassium atom or a sodium atom.

R^{A}, R^{B}, R^{C} and R^{D} are groups which correspond to R^{AF}, R^{BF}, R^{CF} and R^{DF}, respectively. R^{A} to R^{D} are the same groups as R^{AF} to R^{DF}, respectively, or groups which may be perfluorinated and converted by a fluorination reaction to R^{AF} to R^{DF}, respectively. In a case of the latter groups, R^{A} to R^{D} corresponding to R^{AF} to R^{DF} being fluorine atoms, are hydrogen atoms. R^{A} to R^{D} corresponding to R^{AF} to R^{DF} being perfluoro monovalent saturated hydrocarbon groups, are polyfluoro(monovalent hydrocarbon) groups having an arrangement of carbon atoms corresponding to R^{AF} to R^{DF} or monovalent hydrocarbon groups having an arrangement of carbon atoms corresponding to R^{AF} to R^{DF}. Further, R^{A} to R^{D} corresponding to R^{AF} to R^{DF} being perfluoro(partially chlorinated monovalent hydrocarbon) groups, are polyfluoro(partially chlorinated monovalent hydrocarbon) groups having an arrangement of carbon atoms corresponding to R^{AF} to R^{DF} or (partially chlorinated)monovalent hydrocarbon groups having an arrangement of carbon atoms corresponding to R^{AF} to R^{DF}. Each of the hydrocarbon groups of R^{A} to R^{D} may be a saturated group or an unsaturated group, preferably a saturated group.

In a case where R^{AF} is -CFClCF₂Cl, R^{A} may, for example, be -CHClCH₂Cl, -CFClCH₂Cl, -CFClCHFCl, -CHClCHFCl, -CHClCF₂Cl, -CCl=CHCl or -CCl=CFCl. In a case where R^{AF} is -C₄F₉₋ₛClₛ (wherein s is as defined above), R^{A} is -C₄HₛF₉₋₂ₛClₛ (wherein s is as defined above, t is an integer of from 0 to (9-s), preferably (9-s).

R^{EF} is a perfluoro(monovalent saturated hydrocarbon) which may contain an etheric oxygen atom. The number of carbon atoms of R^{EF} is preferably from 2 to 10. R^{EF} is preferably a perfluoroalkyl group or a perfluoroalkyl group containing an etheric oxygen atom, particularly preferably -CF₂CF₃, -CF(CF₃)₂, -(CF₂)₃F, -CF(CF₃)O(CF₂)₃F or -CF(CF₃)OCF₂CF(CF₃)O(CF₂)₃F.

When the compound (1) is produced from the compound (5) in accordance with the above process, it is preferred that the compound (5) corresponding to the structure of the desired compound (1) is suitably prepared and used. The compound (5) is a compound essentially having three partial structures, namely, a 1,3-dioxolane structure, an ester structure and a structure having a group containing chlorine atoms.

With regard to the preparation of the compound (5), it is preferred that the following reaction (a), the following reaction (b) and the following reaction (c) to form the three structures, respectively, are carried out in an optional order by using known compounds, provided that in a case where a compound having one or two of such partial structures can preliminarily be obtained, the reaction step for formation of such partial structures can be omitted.
Reaction (a): reaction for formation of a 1,3-dioxolane structure
Reaction (b): reaction for formation of an ester structure
Reaction (c): reaction for formation of a structure having chlorine atoms or a group having chlorine atoms

The reaction (a) is preferably the following method (a-1) or the following method (a-2).
Method (a-1): a method of obtaining the following compound (50) by reacting the following compound (6A) and the following compound (7) in the presence of an acid catalyst and an orthoacid ester. 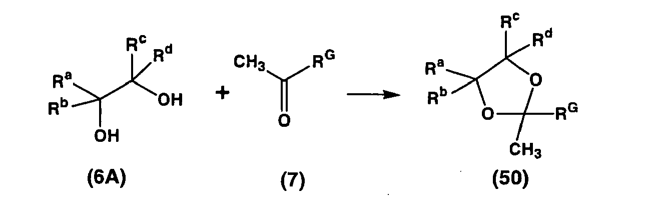
Method (a-2): a method of obtaining the compound (50) by reacting the following compound (6B) and the compound (7) in the presence of an acid catalyst. 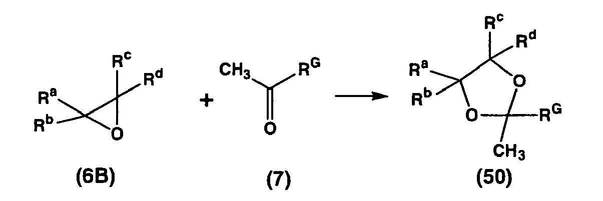 In the formulae, R^{a} to R^{d} correspond to R^{A} to R^{D} of the compound (5), i.e. R^{a}, R^{b}, R^{c} and R^{d} are groups which correspond to R^{A}, R^{B}, R^{C} and R^{D}, respectively, and such groups are the same groups as R^{A} to R^{D} or groups convertible to R^{A} to R^{D}. R^{G} is -CH₂OR^{EF} (wherein R^{EF} is as defined above) or a group convertible to -CH₂OR^{EF} (for example, -CH₃, -CH₂CH₃, -CH₃ or -CH₂OH). For example, in a case of preparing the compound (5) in which R^{A} is a partially chlorinated monovalent saturated hydrocarbon group, R^{a} of the compound (50) is preferably a group convertible to R^{A} by chlorination or the same group as R^{A}.

The compound (50) is the same compound as the compound (5) or a compound readily convertible to the compound (5).

The compound (6A) may, for example, be CH₃CH(OH)CH₂OH, CH₂ClCH(OH)CH₂OH, CH₃CH₂CH(OH)CH₂OH, CH₃CH(OH) CH (OH) CH₃, CH₃CH₂CH₂CH(OH) CH₂OH, CH₃CH₂CH(OH)CH(OH)CH₃, CH₂=CHCH(OH)CH₂OH, CH₃CH₂CH₂CH₂CH(OH) CH₂OH, C(CH₃)₃CH(OH) CH₂OH or CH₂=CHCH(OH)CH(OH)CH=CH₂.

The compound (6B) may be an epoxy compound obtained by oxidation of ClCH₂CH=CHCH₂Cl, CH₂=CHCHClCH₂Cl, CH₂=C(CH₂Cl)₂ or the like to have a -CH=CH- moiety converted into an epoxy structure.

As the compound (7), the following compounds may be mentioned.
CH₃COCH₂OCOCF(CF₃)O(CF₂)₃F, CH₃COCH₂OH, CH₃COCH₃ and CH₃COCH₂CH₃.

The acid catalyst may be an inorganic acid such as hydrochloric acid or sulfonic acid, a Lewis acid such as titanium tetrachloride, boron trifluoride etherate, aluminum chloride or zinc chloride, a benzfluorosulfonate polymer, or such a polymer in a beads form. Further, such an acid catalyst may be a porous nanocomposite having such a polymer supported on amorphous silica.

The orthoacid ester is not particularly limited and may, for example, be HC (OCH₃)₃, HC (OC₂H₅)₃, CH₃C(OCH₃)₃ or CH₃C(OC₂H₅)₃.

The amount of the compound (7) to the compound (6A) in the reaction (a-1) is preferably from 1.0 to 1.5 times by mol, and the amount of the acid catalyst is preferably from 0.1 to 1.0 time by mol. Further, the amount of the orthoacid ester is preferably from 1.0 to 1.5 times by mol. Such a reaction may be carried out in the presence or absence of a solvent, but it is preferably carried out in the absence of a solvent, in view of the volume efficiency. The lower limit of the reaction temperature is preferably -10°C, and the upper limit is preferably the boiling point of the compound (6A) or the boiling point of the compound (7) whichever is lower.

The amount of the compound (7) to the compound (6B) in the reaction (a-2) is preferably from 1.0 to 1.5 times by mol. The amount of the acid catalyst to the compound (6B) is preferably from 0.1 to 1.0 time by mol. The amount of the orthoacid ester to the compound (6B) is preferably from 1.0 to 1.5 times by mol. The reaction is preferably carried out in the same manner as in the case of the reaction (a-1).

For the reaction (b), a method of a conventional esterification reaction or an acetal exchange reaction may be employed.

The esterification reaction may be a method of obtaining the following compound (51-1) by reacting the following compound (50-1) (wherein R^{a} to R^{d} are as defined above) with the compound represented by the formula R^{EF}-COF (wherein R^{EF} is as defined above) or a method of obtaining the following compound (51-1) by reacting the following compound (7-1) with the compound represented by the formula R^{EF}-COF (wherein R^{EF} is as defined above).

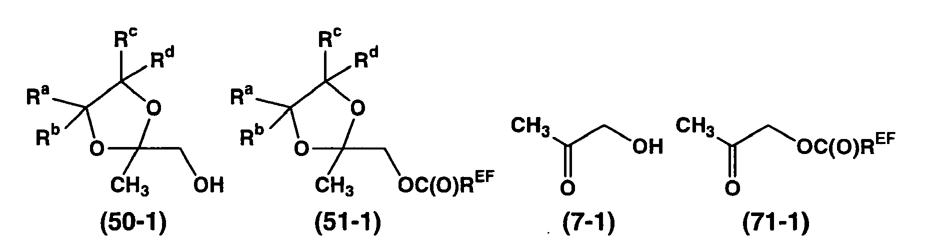

Such reactions can be carried out in accordance with conventional methods (for example, WO02/10106).

The acetal exchange reaction may be a method of reacting the following compound (50-2) or the following compound (50-3), with a compound represented by CH₃COCH₂OCOR^{EF} (wherein R^{EF} is as defined above) (wherein R^{a} to R^{d} in the formulae are as defined above).

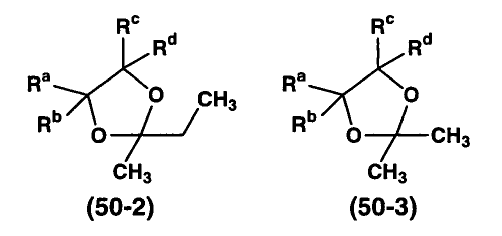

The reaction (c) may be a reaction of chlorination by means of an optional chlorinating agent. Such a chlorinating agent is preferably chlorine. For example, in order to introduce chlorine atoms into a -CH=CH₂ moiety, chlorine is used as the chlorinating agent to convert it into -CHClCH₂Cl. Further, such a moity can similarly be converted into -CHClCH₃ and/or -CH₂CH₂Cl by changing the chlorine to hydrogen chloride. Such chlorine or hydrogen chloride is preferably used in an amount of 1 to 2 times by mol based on the theoretical amount. The reaction temperature is preferably from -78°C to +25°C.

In a case of introducing chlorine atoms into an alkyl group moiety, at least one of hydrogen atoms in the group may be substituted by a chlorine atom by using chlorine as a chlorinating agent to convert the alkyl group to a chloroalkyl group. It is preferred that such a reaction is carried out under irradiation with ultraviolet rays and/or heating. Such chloroalkyl groups produced may usually be at least two groups having different number and/or positions of chlorine atoms introduced, and therefore, such products may be separated and purified after the chlorination reaction as the case requires, or may be used as the mixture.

In order to introduce chlorine atoms into a group having hydroxyl groups, hydrogen chloride or phosphorus trichloride may be reacted thereto as a chlorinating agent, to convert such hydroxyl group moieties to chlorine atoms.

As specific processes for the production of the compound (5), the following two processes may be mentioned.

### PROCESS 1

A process for producing the following compound (51-11) by reacting the following compound (6A-1) with CH₃COCH₂OH to prepare the following compound (50-11), and then reacting the compound (50-11) with R^{EF}-COF (wherein R^{EF} is as defined above). Further, in a case where it is desired to introduce chlorine atoms into the compound (51-11), a process of reacting the compound (51-11) with a chlorinating agent: 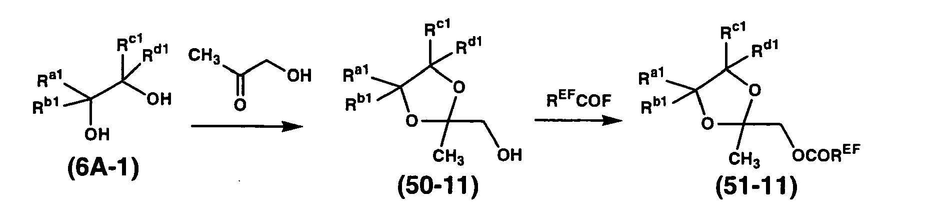 wherein R^{a1}, R^{b1}, R^{c1} and R^{d1} correspond to R^{A}, R^{B}, R^{C} and R^{D}, respectively, and R^{a1} to R^{d1} are the same groups as R^{A} to R^{D}, or groups convertible to the corresponding R^{A} to R^{D} by a chlorination reaction. R^{a1} to R^{d1} being the latter groups, are groups having the number of chlorine atoms smaller than the corresponding R^{A} to R^{D}, groups containing no chlorine atoms, or hydrogen atoms.

### PROCESS 2

A process for producing the following compound (51-11) by reacting the following compound (6B-1) with CH₃COCH₃ to prepare the following compound (50-31), followed by an acetal exchange reaction of the compound (50-31) with separately prepared CH₃COCH₂OCOR^{EF} (wherein R^{EF} is as defined above). Further, in a case where it is desired to introduce chlorine atoms into the compound (51-11), a process for reacting the compound (51-11) with a chlorinating agent (wherein R^{a1} to R^{d1} in the formula are as defined above).

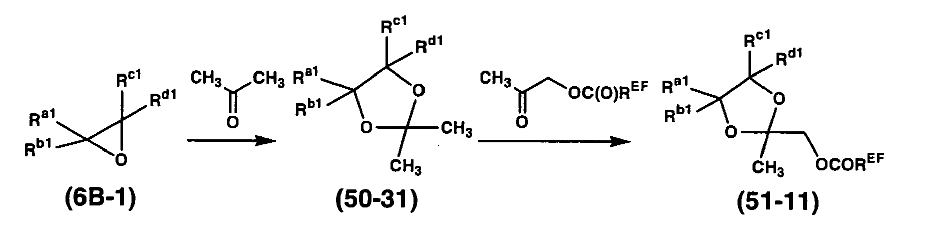

The reaction for producing the compound (1) by thermal decomposition of the compound (3) can be carried out in accordance with the method disclosed in WO03/037885. The thermal decomposition of the compound (3) may be carried out by a reaction in one step, or may be carried out by a reaction in two steps which comprises reacting the compound (3) with an alkali metal hydroxide (potassium hydroxide or sodium hydroxide) to convert it to the compound (3A), followed by the thermal decomposition. Such a reaction may also be carried out in accordance with the method disclosed in WO03/037885.

A compound to be used for the production process for the compound (1) of the present invention may be a known compound or the following novel compound (wherein s, X¹⁰ and X¹ are as defined above, and the group represented by the formula -C₄H₉₋ₛClₛ is a linear group).

### EXAMPLES OF THE COMPOUND (50-1)

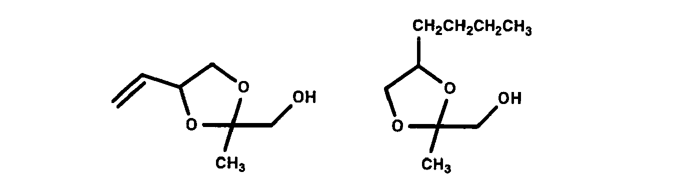

### EXAMPLES OF THE COMPOUND (51-1)

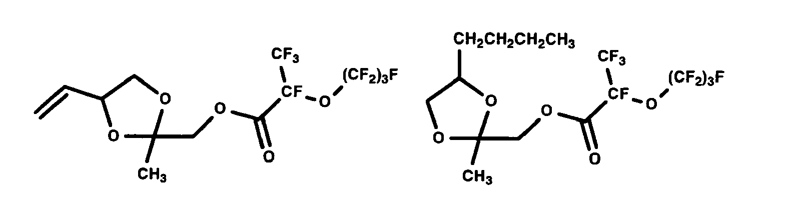

### EXAMPLES OF THE COMPOUND (5)

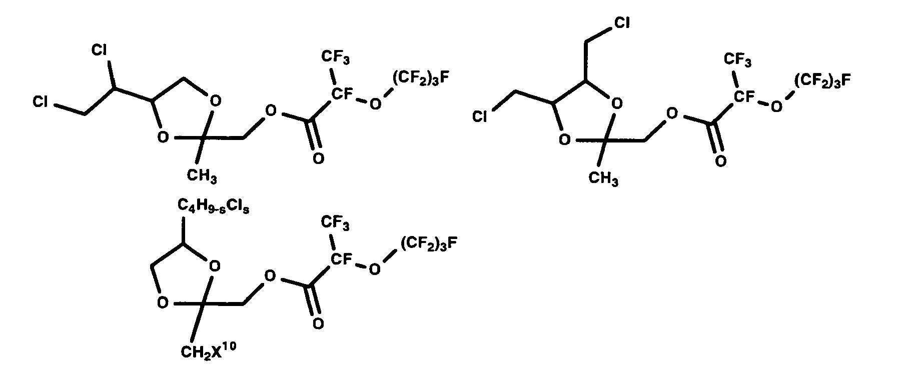

### EXAMPLES OF THE COMPOUND (4)

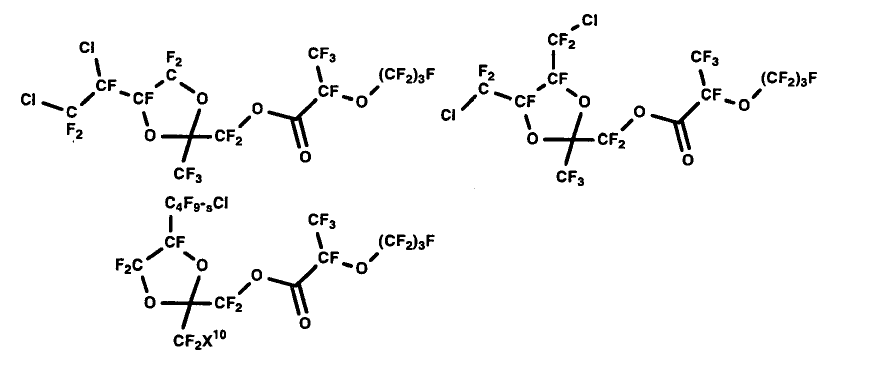

### EXAMPLES OF THE COMPOUND (3)

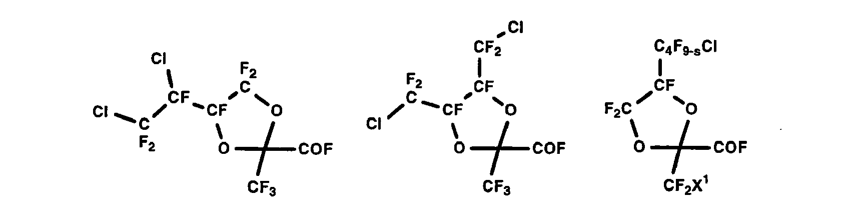

Since the compound (1) of the present invention has a polymerizable unsaturated group, the polymer obtained by polymerizing such a compound is a fluoropolymer comprising the following monomer units (2) (hereinafter referred to as the fluoropolymer (2)).

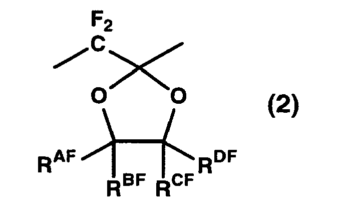

The monomer units (2) can be obtained as monomer units obtained by polymerizing the compound (1). The fluoropolymer (2) is preferably a polymer obtained by polymerizing one type of the compound (1), a polymer obtained by polymerizing at least two types of the compound (1), or a polymer obtained by polymerizing at least one type of the compound (1) and at least one type of another monomer to be polymerized with the compounds (1).

As a specific example for the monomer units (2), a fluoropolymer comprising monomer units (2a) obtained by polymerizing the above compound (1a), a fluoropolymer comprising monomer units (2b) obtained by polymerizing the above compound (1b), and a fluoropolymer comprising monomer units (2c) obtained by polymerizing the above compound (1c) may be mentioned (wherein s is as defined above, and the group represented by the formula -C₄H₉₋ₛClₛ is a linear group).

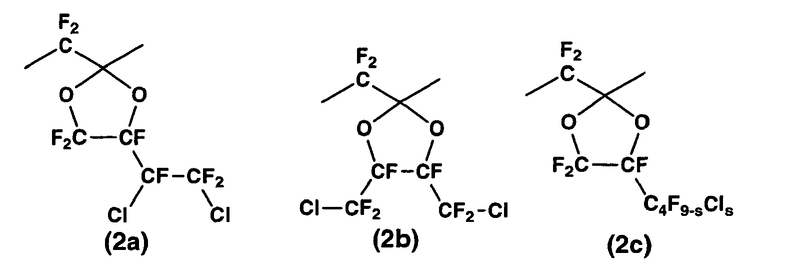

In a case where the fluoropolymer (2) contains monomer units of another monomer, the ratio of the monomer units (2) based on the total monomer units in the fluoropolymer (2), is preferably from 0.1 to 99.9 mol%, particularly preferably from 40 to 75 mol%. The ratio of the monomer units formed by polymerization of another monomer is preferably from 0.1 to 99.9 mol%, particularly preferably from 60 to 25 mol%.

Such another monomer to be polymerized with the compound (1) is not particularly limited, and may be a perfluoroolefin such as tetrafluoroethylene or hexafluoropropylene, a perfluoro(vinyl ether) such as perfluoro(alkyl vinyl ether), a cyclopolymerizable perfluorodiene such as perfluoro (3-butenyl vinyl ether), perfluoro(allyl vinyl ether) or perfluoro(3,5-dioxa-1,6-heptadiene), a fluorinated cyclic olefin such as perfluoro(2,2-dimethyl-1,3-dioxol), perfluoro(1,3-dioxol), perfluoro(4-methoxy-1,3-dioxol), a perfluoro(2-methylene-1,3-dioxolane) containing no fluorine atoms such as perfluoro(2-methylene-1,3-dioxolane), a chlorofluoroolefin such as chlorotrifluoroethylene, a partially fluorinated olefin such as trifluoroethylene, vinylidene fluoride, vinyl fluoride, a (perfluoroalkyl)ethylene, a (perfluoroalkyl)propene, or a hydrocarbon type olefin such as ethylene or propylene.

Such another monomer is preferably selected from perfluorinated compounds, and in view of light transmittance, it is particularly preferably a perfluoroolefin,a cyclopolymerizable perfluorodiene, a perfluorovinyl ether or a perfluoro(2-methylene-1,3-dioxolane) containing no chlorine atoms.

The number average molecular weight of the fluoropolymer (2) is preferably from 5,000 to 5,000,000, particularly preferably from 10,000 to 3,000,000.

A process for the production of the fluoropolymer (2) is preferably a process of reacting the compound (1) by radical polymerization in the presence of a radical polymerization initiator. The radical polymerization reaction can be carried out by bulk polymerization wherein the compound (1) is polymerized as it is, solution polymerization wherein it is polymerized in a solution, suspension polymerization wherein it is polymerized in an aqueous medium in the presence or absence of an appropriate organic solvent, or emulsion polymerization wherein it is polymerized in an aqueous medium in the presence of an emulsifier.

The radical polymerization initiator may be a radical polymerization initiator to be used for a usual radical polymerization, such as an azo compound, an organic peroxide or an inorganic peroxide. As a specific radical initiator, an azo compound such as 2,2'-azobis(2-amidinopropene) dihydrochloride, 4,4'-azobis(4-cyanopentanoic acid), 2,2'-azobis(4-methoxy-2,4-dimethyl valeronitrile) or 1,1'-azobis(1-cyclohexane carbonitrile), an organic peroxide such as diisopropyl peroxydicarbonate, benzoyl peroxide, perfluorononanoyl peroxide, methyl ethyl ketone peroxide, diisopropyl peroxide, (C₃F₇COO)₂, (C₆F₅COO)₂ or ((CH₃)₃CO)₂, or an inorganic peroxide such as K₂S₂O₈ or (NH₄)₂S₂O₈, may be mentioned.

The polymerization temperature is not particularly limited, and it is preferably from 0 to 200°C, particularly preferably from 30 to 100°C. Further, the polymerization pressure may be elevated pressure, reduced pressure or atmospheric pressure, and it is practically preferably from -0.1 to +9 MPa (gage pressure), particularly preferably from -0.1 to +4 MPa (gage pressure).

The fluoropolymer (2) of the present invention may be used for various applications as it is, or may be used for various applications after a chemical conversion.

Such applications of the fluoropolymer (2) may, for example, be an optical material such as an optical fiber material (a core material or a clad material for an optical fiber), an optical waveguide material (a core material or a clad material for an optical waveguide material) or a 45° mirror material for a 90° optical path conversion of an optical/electrical circuit board, a material for electronic components such as an interlayer dielectric film (for example, for a semiconductor element, for a liquid crystal display panel or for a multilayer wiring board), a buffer coating film, a passivation layer, an α-ray encapsulation layer, an element encapsulation medium, an interlayer dielectric film for high-density substrates, a protective film for high frequency elements (for example, an RF circuit element, a GaAs element and an InP element), and a film material.

Especially, the fluoropolymer (2) is a non-crystalline fluoropolymer having excellent mechanical strength, transparency and thermal stability and having a high refractive index, and it is thus useful as an optical material, and particularly useful as an optical waveguide material. For example, an optical waveguide using the fluoropolymer (2) for a clad material and using a low refractive index material for a core material, has a large difference in refractive index between the core and the clad and has a large number of numerical apertures, and it thus has an advantage such that the light introducing efficiency is high. Further, the optical waveguide has a small curvature radius, and its optical component can be downsized, and therefore such an optical waveguide can be used as a bent optical waveguide. Further, the fluoropolymer (2) of the present invention has high transparency and has excellent adhesiveness to various substrates, whereby it can be used also for an adhesive for optics such as an adhesive for bonding optical paths to connect optical paths.

The fluoropolymer (2) of the present invention may be used as processed into various shapes depending upon the particular application.

For example, in a case where the fluoropolymer (2) is to be used in a film form, first, the fluoropolymer (2) is preferably dissolved in an organic solvent to be a solution composition. The organic solvent is preferably a fluorinated organic solvent, and it may, for example, be a chlorohydrocarbon such as trichlorofluoromethane or trichlorotrifluoroethane, or a hydrohydrochlorocarbon such as 1,1-dichloro-1-fluoroethane, 2,2,2-trifluoro-1,1-dichloroethane, dichloropentafluoropropane or hexafluoro-1,1,3,4-tetrachlorobutane.

The amount of the fluoropolymer (2) of the present invention contained in the solution composition is preferably adjusted depending upon thickness of a coating film, and is preferably from 0.01 to 20 mass%, particularly preferably from 0.1 to 15 mass% based on the solution composition.

When a film is to be formed from the solution composition, it is preferred that the solution composition is applied on a substrate surface and then dried to form a coating film made of the fluoropolymer (2) on the substrate surface. The coating film may be used for the above applications as it is formed on the substrate surface, or the coating film may be peeled from the substrate surface and then used for the above applications. The thickness of the coating film is changed depending upon the applications, and is usually from 0.001 to 1,000 µm. The method of coating the substrate surface may, for example, be roll coating, casting, dip coating, spin coating, cast on water, die coating or Langmuir Blodgett. The thickness of the coating film is changed depending upon the applications, and it is usually from 0.001 to 1,000 µm.

### EXAMPLES

Now, the present invention will be described in further detail with reference to Examples. However, it should be understood that the present invention is by no means restricted thereto.

In Examples, CCl₂FCClF₂ will be represented simply by R-113, dichloropentafluoropropane R-225, (t-butyl)methyl ether MTBE, the gas chromatography GC, the size exclusion chromatography GPC, the number average molecular weight Mₙ, the mass average molecular weight M_{w}, and the glass transition point Tg. The pressure will be represented by a gage pressure unless otherwise specified. The purity was determined by the peak area ratio by a gas chromatography analysis.

The molecular weight was measured by a GPC method. Such a measurement was in accordance with the method as disclosed in JP-A-2000-74892. Specifically, a mixed liquid of CClF₂CF₂CHClF and (CF₃)₂CHOH (volume ratio 99:1) was used as the mobile phase, and two columns of (PLgel 5 µm MIXED-C (inner diameter 7.5 mm, length 30 cm)) manufactured by Polymer Laboratories Ltd. were connected in series to obtain a column for analysis. As standard samples for molecular weight measurement, 10 kinds of polymethyl methacrylates having molecular weights of from 1,000 to 2,000,000 and having a molecular weight distribution (M_{w}/Mₙ) of less than 1.17 (manufactured by Polymer Laboratories Ltd.) were used to prepare a calibration curve. The mobile phase flow rate was 1.0 mL/min, the column temperature was 37°C, and an evaporative light scattering detector was used as a detector. Each of Mₙ and M_{w} will be represented by a molecular weight calculated as polymethylmethacrylate. Further, Tg was measured by a DSC method.

### EXAMPLE 1

### EXAMPLE OF PRODUCTION OF COMPOUND (50-1a)

CH₂=CHCH(OH)CH₂OH (114.2 g), HC(OCH₂CH₃)₃ (193.9 g), CH₃COCH₂OH (80.2 g) and an ion exchange resin (4.0 g, manufactured by E.I. du Pont de Nemours and Company, trade name: Nafion SAC-13) were charged into a flask and stirred for 30 minutes while the internal temperature of the flask was kept to be 25°C. Then, the internal temperature of the flask was raised to 80°C, and a low-boiling component was distilled off with stirring for 4 hours to obtain a crude liquid. After filtration of the crude liquid, the filtrate was distilled under reduced pressure, and as a result, a fraction (131.0 g) of (59.9 to 61.2)°C/0.27 kPa (absolute pressure) was obtained. The fraction was analyzed by GC and NMR, and as a result, it was confirmed that the fraction was composed of the following compound (50-1a) and CH₂=CHCH(OH)CH₂OH. The purity of the compound (50-1a) was 85%.

The fraction (118.3 g), HC (OCH₂CH₃)₃ (23.4 g), CH₃COCH₂OH (11.7 g) and an ion exchange resin (0.7 g, trade name: Nafion SAC-13) were charged into a flask and stirred for two hours while the internal temperature of the flask was kept to be 80°C. After filtration of the solution in the flask, MTBE (1 L) and water (30 mL) were added thereto to obtain a double layered liquid. The organic layer of the liquid was separated and then washed four times with water (10 mL), and after dehydration with magnesium sulfate, MTBE was distilled off to obtain a crude liquid. The crude liquid was distilled under reduced pressure, and a fraction of (59.0 to 60.2)°C/0.77 kPa (absolute pressure) (92.0 g) was obtained. The fraction was analyzed by GC and NMR, and as a result, it was confirmed that the compound (50-1a) in a purity of 90% was produced.

¹H-NMR (300.4 MHz, solvent: CDCl₃, standard: TMS) δ (ppm): 1.38, 1.40 (s, 3H), 3.52 to 3.68 (m, 3H), 4.13 to 4.20 (m, 1H), 4.53 to 4.61 (m, 1H), 5.23 to 5.42 (m, 2H), 5.77 to 5.89 (m, 1H).

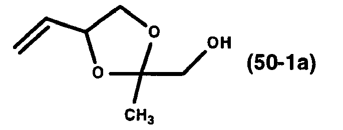

### EXAMPLE 2

### EXAMPLE OF PRODUCTION OF COMPOUND (51-1a)

The compound (50-1a) (103.0 g) obtained in Example 1, (CH₃CH₂)₃N (87.8 g) and R-225 (184.0 g) were charged into a three-necked flask (internal capacity: 1 L). While the internal temperature of the flask was kept to be at most 10°C with stirring, FCOCF(CF₃)O(CF₂)₃F (284.8 g) was dropwise added over a period of 3.5 hours. After completion of the dropwise addition, stirring was further carried out at 25°C for 1 hour, and ice water (500 mL) was added to the flask to obtain a double layered liquid. The lower layer of the liquid was separated and washed two times with water (250 mL), and after dehydration with magnesium sulfate, distillation under reduced pressure was carried out, and as a result, a fraction of (72.1 to 77.9)°C/0.67 kPa (absolute pressure) (156.3 g) was obtained. The fraction was analyzed by GC andNMR, and as a result, it was confirmed that the following compound (51-1a) (156.3 g) in a purity of 97.7% was produced.

¹H-NMR (300.4 MHz, solvent: CDCl₃, standard: TMS) δ (ppm): 1.43, 1.45 (s, 3H), 3.57 to 3.67 (m, 1H), 4.11 to 4.59 (m, 4H), 5.23 to 5.40 (m, 2H), 5.72 to 5.86 (m, 1H).

¹⁹F-NMR (282.7 MHz, solvent: CDCl₃, standard: CFCl₃) δ (ppm): -80.0 (1F), -81.3 (3F), -82.0 (3F), -86.3 (1F), -129.4 (2F), -131.6 (1F).

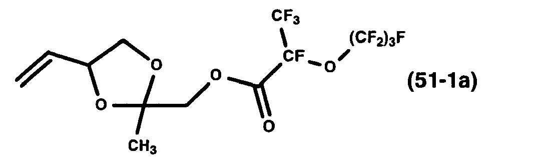

### EXAMPLE 3

### EXAMPLE OF PRODUCTION OF COMPOUND (5a)

The compound (51-1a) (156.3 g) obtained in Example 2 was charged into a three-necked flask (internal capacity: 1 L) equipped with a stirrer and a dry ice condenser, and while the internal temperature of the flask was kept to be at -20°C with stirring, chlorine gas (31.4 g) was blown into the flask over a period of 2.5 hours. Then, the internal temperature of the flask was adjusted to 25°C, and while nitrogen gas was introduced thereinto, stirring was carried out for 1 hour to remove the chlorine gas in the flask. Then, R-225 (300 g) was added to the flask, and then water (100 mL) was added thereto to obtain a double layered liquid. The organic layer of the liquid was separated, and after dehydration with magnesium sulfate, concentrated by an evaporator to obtain a concentrated liquid (207.5 g). The concentrated liquid (70.0 g) was purified by means of a silica gel column chromatography employing R-225 as a developing solvent to obtain a product (44.5 g). The product was analyzed by GC and NMR, and as a result, it was confirmed that the following compound (5a) in a purity of 83% was produced.
¹H-NMR (300.4 MHz, solvent: CDCl₃, standard: TMS) δ (ppm): 1.41, 1.44, 1.46, 1.50 (s, 3H), 3.71 to 4.09 (m, 4H), 4.17 to 4.72 (m, 4H).
¹⁹F-NMR (282.7 MHz, solvent: CDCl₃, standard: CFCl₃) δ (ppm): -79.9 (1F), -81.3 (3F), -82.0 (3F), -86.3 (1F), -129.4 (2F), -131.7 (1F).

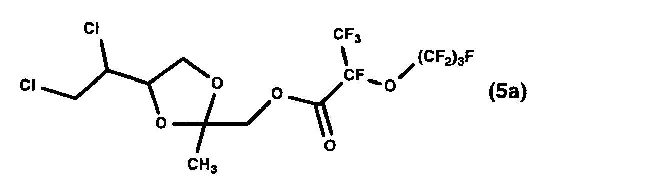

### EXAMPLE 4

### EXAMPLE OF PRODUCTION OF COMPOUND (4a)

Into an autoclave (made of nickel, internal capacity: 500 mL), R-113 (312 g) was charged, stirred and maintained at 25°C. At the gas outlet of the autoclave, a condenser kept to be 20°C, a NaF pellet-packed layer and a condenser kept to be -10°C were installed in series. Further, a liquid-returning line was installed to return the liquid condensed from the condenser kept to be -10°C, to the autoclave. Nitrogen gas was introduced into an autoclave at 25°C for 1 hour, and then, fluorine gas diluted to 20% with nitrogen gas (hereinafter referred to as a 20% diluted fluorine gas) was introduced at a flow rate of 13.43 L/h for 1 hour at 25°C.

Then, while a 20% diluted fluorine gas was introduced at the same flow rate, a solution having the compound (5a) (10.0 g) obtained in Example 3, dissolved in R-113 (120 g), was injected over a period of 4.9 hours, then, while the 20% diluted fluorine gas was introduced at the same flow rate, an R-113 solution having a benzene concentration of 0.01 g/mL was injected in an amount of 9 mL while the temperature was raised from 25°C to 40°C, whereupon the solution inlet of the autoclave and the outlet valve of the autoclave were closed. After the pressure in the autoclave was adjusted to 0.15 MPa, a fluorine gas inlet valve of the autoclave was closed, and stirring was continued for 0.3 hour. Then, while the pressure in the autoclave was kept to be 0.15 MPa and the temperature was kept to be 40°C, an R-113 solution was injected in an amount of 6 mL, whereupon the solution inlet of the autoclave was closed, and stirring was continued for 0.3 hour. Then, the same operation was repeated once. The total amount of benzene injected was 0.21 g, and the total amount of R-113 was 21 mL.

Then, while the 20% diluted fluorine gas was introduced at the same flow rate, stirring was continued for 1 hour. Then, while the pressure in the autoclave was kept to be an atmospheric pressure, nitrogen gas was supplied for 1 hour to obtain a product. The product was analyzed by ¹⁹F-NMR, and as a result, the following compound (4a) was obtained at a yield of 86%.

¹⁹F-NMR (282.7 MHz, solvent: CDCl₃, standard: CFCL₃) δ (ppm): -61.7 to -65.5 (2F), -73.2 to -75.0 (1F), -79.1 to -81.9 (11F), -85.6 to -87.8 (3F), -110.5 to -114.4 (1F), -128.4 to -130.1 (3F), -131.9 (1F).

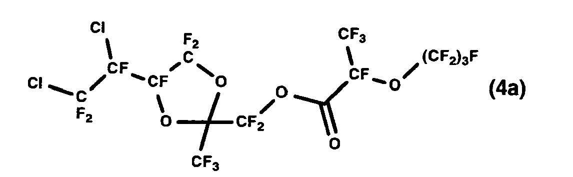

### EXAMPLE 5

### EXAMPLE OF PRODUCTION OF COMPOUND (3a)

KF (1.0 g) was put into a flask, and while the pressure was reduced by a vacuum pump, its interior was heated for 30 minutes by a heat gun and cooled to 25°C. While an interior of the flask was kept at an atmospheric pressure with nitrogen gas, the compound (4a) (59.0 g) obtained in Example 4 was put thereto, and then stirred for 2 hours while an internal temperature of the flask was kept to be 40°C. Then, while the internal temperature of the flask was kept to be 90°C, the low-boiling component in the flask was distilled off, and then KF was filtered off to obtain a product (42.8 g). The product was analyzed by GC and NMR, and as a result, it was found that the following compound (3a) having a purity of 70.7% was produced.

¹⁹F-NMR (282.7 MHz, solvent: CDCl₃, standard: CFCl₃) δ (ppm): 25.3 to 24.3 (1F), -61.3 to -65.0 (2F), -73.6 to -76.3 (1F), -80.7 to -81.1 (3F), -81.3 to -84.7 (1F), -111.8 to -117.5 (1F), -128.2 to -129.4 (1F).

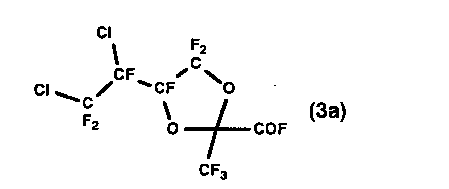

### EXAMPLE 6

### EXAMPLE OF PRODUCTION OF COMPOUND (3Aa)

Ethanol (10.5 g) was put into a flask in an atmosphere of nitrogen gas, and while the internal temperature of the flask was kept to be below 10°C, the compound (3a) (42.7 g) obtained in Example 5 was dropwise added over a period of 1 hour, and then stirring was carried out for 1 hour. After the compound (3a) was confirmed to be disappeared by a GC analysis, water (5 mL) was added to the flask to obtain a double layered liquid. The organic layer of the liquid was washed three times with water (5 mL), and after dehydration with magnesium suflate, put into a flask charged with methanol (4.0 mL). Then, while the internal temperature of the flask was kept to be below 0°C in a nitrogen gas atmosphere, a methanol solution (32.5 g) containing 15 mass% of KOH was dropwise added thereto, and then a few drops of a methanol solution containing 1 mass% of phenol phthalein was dropwise added thereto. The solution in the flask was found to be colored blue. Then, the methanol solution containing 15 mass% of KOH was dropwise added thereto until the solution in the flask was colored pink. The total amount of the methanol solution containing 15 mass% of KOH dropped was 46.8 g. The solution in the flask was concentrated and vacuum-dried at 60°C for 17 hours, and the solid material thus obtained was pulverized by mortar and vacuum-dried at 60°C for 24 hours, and as a result, the following compound (3Aa) (41.9 g) was obtained.

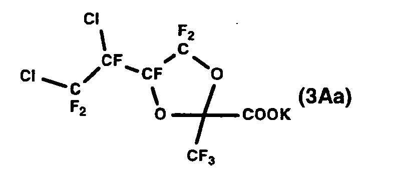

### EXAMPLE 7

### EXAMPLE OF PRODUCTION OF COMPOUND (1a)

The compound (3Aa) (20.5 g) obtained in Example 6 was put into a flask. At the outlet of the flask, a trap kept to be -78°C and a vacuum pump was connected in order. When the internal pressure of the flask was reduced to 0.7 kPa (absolute pressure) and then the flask was heated, distillate was gathered in a trap at the time when the internal temperature of the flask was in the vicinity of 190°C. The flask was further heated to 257°C and reaction was continued for 3.5 hours. The distillate in the trap was the following compound (1a) (14.4 g) having a GC purity of 48.4%. The distillate was distilled under reduced pressure to obtain a fraction (2.7 g) of 28°C/2.1 kPa (absolute pressure). The fraction was analyzed by GC and NMR, and as a result, it was confirmed that the compound (1a) having a purity of 92.7% was formed.

¹⁹F-NMR (282.6 MHz, solvent: CDCl₃, standard: CFCl₃) δ (ppm): -61.7 to -65.3 (2F), -78.3 to -79.9 (1F), -87.2 to -89.0 (1F), -117.4 to -121.0 (1F), -125.4 to -126.0 (1F), -126.7 to -127.2 (1F), -129.3 to -130.3 (1F).

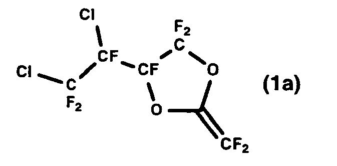

### EXAMPLE 8

### EXAMPLE OF HOMOPOLYMERIZATION OF COMPOUND (1a)

Into a glass ampoule, the compound (1a) (1.3 g) obtained in Example 7, ((CH₃)₂CHOCOO)₂ (0.065 g) and R-225 (1.0 g) were charged and freeze-deaerated, followed by sealing. The resulting mixture was heated at 50°C for 20 hours in an oven and the polymerization reaction was carried out to obtain a solid material. The solid material was dissolved in R-225, and purified by reprecipitation method using hexane (20 mL). Further, washing with hexane was repeated two times, and then vacuum drying was carried out at 50°C for 16 hours to obtain a polymer as a white powder (0.5 g).

The polymer was analyzed by ¹⁹F-NMR, and as a result, the signal based on the double bond of the compound (1a) completely disappeared, whereby it was found to be a polymer made of the following monomer units (2a). Further, Mₙ of the polymer was 8.8×10⁴, M_{w} was 1.26×10⁵, and Tg was 179°C.

Then, the polymer was dissolved in hexafluoro-1,1,3,4-tetrachlorobutane to obtain 10 mass% of a solution composition. Using a PTFE sheet as a substrate, such a solution composition was applied on a substrate by casting, and then dried at 150°C for 1 hour to form a coating film on a surface of the substrate. The coating film was peeled from the surface of the substrate to obtain a thin film having a thickness of 110 µm. The thin film was measured by an Abbe refractometer, and as a result, the refractive index was found to be 1.393.

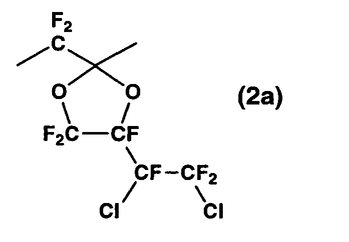

### EXAMPLE 9

### EXAMPLE OF COPOLYMERIZATION OF COMPOUND (1a)

Into a glass ampoule, the compound (1a) (1.3 g) obtained in Example 7, CF₂=CFOCF₂CF₂CF=CF₂ (1.13 g) and (C₆F₅COO)₂ (0.012 g) were put and freeze-deaerated, followed by sealing. The mixture was heated at 65°C for 18.5 hours in an oven and polymerization reaction was carried out to obtain a solid material. The solid material was dissolved in R-225, and purified by reprecipitation using hexane (70 mL). Further, after washing was repeated two times with hexane, vacuum-drying was carried out at 60°C for 16 hours to obtain a polymer as a white powder (1.7 g).

### EXAMPLE 10

### EXAMPLE OF PRODUCTION OF COMPOUND (6B-1a)

Into a flask (internal capacity: 100 mL), ClCH₂CH=CHCH₂Cl (10.0 g) and anhydrous methylchloride (10 mL) were charged, and m-chloroperbenzoic acid (25.0 g) was dividedly added every 30 minutes in five times with stirring at 25°C for 72 hours to react them. The reaction solution obtained was analyzed by GC, and as a result, it was confirmed that ClCH₂CH=CHCH₂Cl disappeared. The same reaction was separately carried out. The reaction solutions obtained in the reactions in two times were collected in a flask, and 100 g/L of a Na₂S₂O₃ aqueous solution (100 mL) was dropwise added to the flask to obtain a double layered liquid. The organic layer was separated and washed with a saturated sodium hydrogen carbonate aqueous solution and further with water, and then dehydrated with magnesium sulfate. Further, the dehydrate was concentrated by an evaporator to obtain a concentrate (22.1 g). The concentrate was analyzed by GC and NMR, and as a result, it was confirmed that the following compound (6B-1a) having a purity of 97.5% was formed.

¹H-NMR (300.4 MHz, solvent: CDCl₃, standard: TMS) δ (ppm): 3.22 (m, 2H), 3.60 (m, 4H).

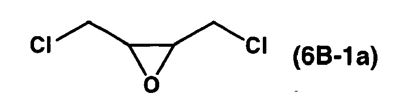

### EXAMPLE 11

### EXAMPLE OF PRODUCTION OF COMPOUND (71a)

Into a flask (internal capacity: 300 mL) under a nitrogen gas atmosphere, CH₃COCH₂OH (50.0 g), R-225 (81.6 g) and NaF (85.3 g) were put, and stirring was continued while the internal temperature of the flask was kept to be at most 10°C. FCOCF(CF₃)OCF₂CF₂CF₃ (213.3 g) was dropwise added thereto over a period of two hours, and then the internal temperature of the flask was returned to 25°C and stirring was carried out for 12 hours. Into a filtrate obtained by pressure filtration of a solution in the flask, a saturated sodium hydrogen carbonate aqueous solution (200 mL) was added to obtain a double layered solution. The organic layer was separated and washed with water (200 mL) and dehydrated with magnesium sulfate and then concentrated by an evaporator, to obtain a concentrate (230.7 g). In the concentrate, the following compound (71a) was contained, and a GC purity of the compound (71a) was 87.2%. The reaction was separately carried out under the same conditions, whereby a concentrate (96.0 g) having a GC purity of 96.5% was obtained. The concentrate obtained by the reaction twice was distilled under reduced pressure, and as a result, a fraction (281.3 g) of 67 to 71°C/1.06 kPa (absolute pressure) was obtained. The fraction was analyzed by GC and NMR, and as a result, it was confirmed that the compound (71a) having a purity of 97.9% was formed.

¹H-NMR (300.4 MHz, solvent: CDCl₃, standard: TMS) δ (ppm): 2.21 (s, 3H), 4.91 (q, 16.5 Hz, 2H).

¹⁹F-NMR (282.6 MHz, solvent: CDCl₃, standard: CFCl₃) δ (ppm): -79.6 to -80.3 (1F), -81.7 to -81.8 (3F), -82.4 to -82.5 (3F), -86.5 to -87.0 (1F), -130.1 to -130.2 (2F), -132.8 (1F).

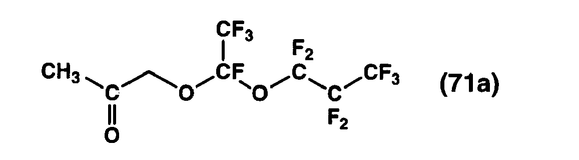

### EXAMPLE 12

### EXAMPLE OF PRODUCTION OF COMPOUND (5b)

Into a flask (internal capacity: 200 mL) under a nitrogen gas atmosphere, boron trifluoride etherate (2.0 g), the compound (6B-1a) (10.1 g) obtained in Example 10 and an anhydrous acetone (39.0 g) were charged, and stirred at 25°C for 4 hours, and then stirring was further carried out at 30°C for 2.5 hours. Then, into the flask, boron trifluoride etherate (1.0 g) was added, and stirring was carried out for 12 hours.

Then, a solution made of the compound (71a) (33.0 g) obtained in the same manner as in Example 10 and an anhydrous toluene (51.0 g), was added to the flask at 25°C. Then, distillation was carried out at 35°C and under 6.5 kPa (absolute pressure) to obtain a reaction crude liquid in which the solution in the flask was distilled off. The reaction crude liquid was analyzed by GC, and as a result, it was confirmed that the following compound (50-3a) as a reaction intermediate remains, and therefore, boron trifluoride etherate (0.8 g) was further added to the distilled liquid to obtain a reaction solution having acetone distilled off under the condition of (35 to 40)°C/6.5 to 13 kPa (absolute pressure).

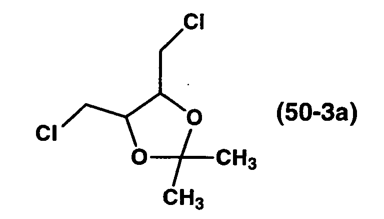

The organic layer of the double layered liquid obtained by adding a saturated sodium hydrogen carbonate aqueous solution (100 mL) and MTBE (100 mL) to the reaction solution, was separated and washed with water, and after dehydration with magnesium sulfate, concentrated by an evaporator to obtain a concentrate (37.3 g). In the concentrate, the following compound (5b) was contained in a GC purity of 34.8%. The concentrate (36.9 g) was purified by a silica gel column chromatography using R-225 as a developing solvent to obtain an eluent (17.1 g). The eluent was analyzed by GC and NMR, and as a result, it was confirmed that the compound (5b) was contained in a purity of 75.5%. Further, such aneluent was distilled under reduced pressure, and as a result, a fraction (8.2 g) of (64.6 to 102)°C/0.98 to 1.03 kPa (absolute pressure) was obtained. The fraction was analyzed by GC and NMR, and as a result, it was confirmed that the compound (5b) was contained in a purity of 97.9%.

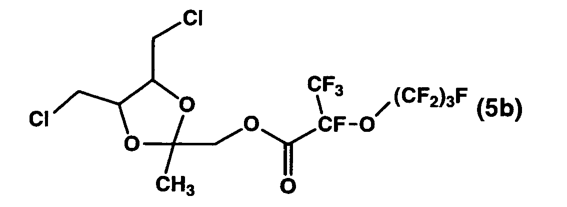

### EXAMPLE 13

### EXAMPLE OF PRODUCTION OF COMPOUND (4b)

The same autoclave as in Example 4 was prepared. After introducing nitrogen gas into an autoclave at 25°C for 1 hour, a 20% diluted fluorine gas was introduced at 25°C for 1 hour at a flow rate of 10.8 L/h. The pressure in the autoclave showed atmospheric pressure.

Then, while a 20% diluted fluorine gas was introduced at the same flow rate, a solution prepared by dissolving the compound (5b) (4.0 g) obtained in Example 12 in R-113 (62.0 g), was injected over a period of 2.6 hours. Further, while fluorine gas diluted to 20% was introduced at the same flow rate, a R-113 solution containing 0.01 g/mL of a benzene (hereinafter referred to as R-113 solution) was injected in an amount of 9 mL while the temperature was raised from 25°C to 40°C, whereupon the solution inlet of the autoclave and the outlet valve of the autoclave were closed. When the pressure in the autoclave was kept to be 0.15 MPa, the fluorine gas inlet valve of the autoclave was closed, and stirring was continued for 15 minutes. Then, while the internal pressure of the autoclave was kept to be 0.15 MPa and the internal temperature of the autoclave was kept to be 40°C, the R-113 solution was injected in an amount of 6 mL, whereupon the solution inlet of the autoclave was closed, and stirring was continued for 15 minutes. Further, the same operation was repeated once. The total amount of benzene injected was 0.21 g and the total amount of the R-113 solution injected was 21 mL.

Further, the 20% diluted fluorine gas was injected at the same flow rate, and stirring was continued for 1 hour. Then, the internal pressure of the autoclave was kept to be at an atmospheric pressure, and nitrogen gas was supplied for 1 hour to obtain a product. The product was quantified by ¹⁹F-NMR, and as a result, it was confirmed that the following compound (4b) was produced at a yield of 82%.

¹⁹F-NMR (282.7 MHz, solvent: CDCl₃, standard: CFCl₃) δ (ppm): -65 to 69 (m, 4F), -80 (m, 4F), -82 (m, 6F), -86 (m, 3F), -106 to -116 (m, 2F), -130 (m, 2F), -132 (m, 1F).

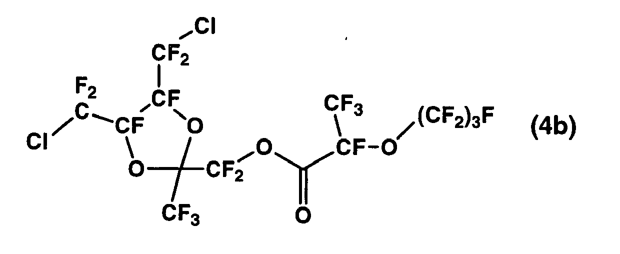

### EXAMPLE 14

### EXAMPLE OF PRODUCTION OF COMPOUND (3b)

KF is put into a flask, and while the pressure was reduced by a vacuum pump, its interior is heated for 30 minutes by a heat gun and cooled to 25°C. While the interior of the flask is kept to be atmospheric pressure by nitrogen gas, the compound (4b) obtained in the same manner as in Example 12 is charged thereto, and the internal temperature of the flask is kept to be 40°C, followed by stirring for 2 hours. Then, the internal temperature of the flask is kept to be 90°C and the low boiling point component in the flask is distilled off, whereupon KF is filtered off to obtain the following compound (3b).

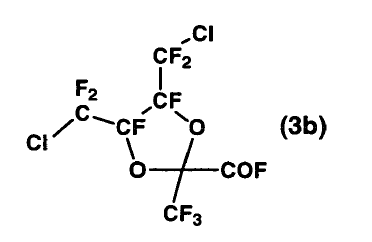

### EXAMPLE 15

### EXAMPLE OF PRODUCTION OF COMPOUND (3Ab)

Methanol is charged into a flask under a nitrogen gas atmosphere, and while the internal temperature of the flask is kept to be at most 10°C, the compound (3b) obtained in the same manner as in Example 13 is dropwise added over a period of 1 hour, followed by stirring for 1 hour. After the compound (3b) is confirmed to be disappeared by a GC analysis, water is added to the flask to obtain a double layered liquid. The organic layer of the liquid is washed three times with water and dehydration with magnesium sulfate in order, and then charged into a flask supplied with methanol. The interior of the flask is maintained such that the internal temperature of the flask is kept to be at most 0°C under a nitrogen gas atmosphere, and then a few drops of a methanol solution containing 1 mass% of a phenol phthalein is added thereto. The solution in the flask is colored blue. Then, a methanol solution containing 1 mass% of KOH is dropwise added to the solution until the reaction liquid is colored pink. Such a solution is concentrated, and the solid material obtained by vacuum drying at 60°C is pulverized by mortar, and further vacuum-dried at 60°C for 24 hours to obtain the following compound (3Ab).

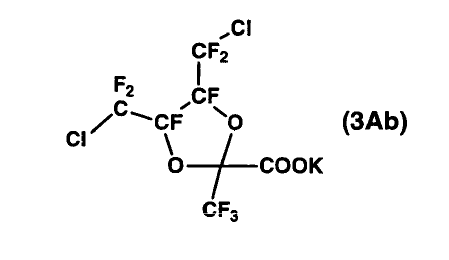

### EXAMPLE 16

### EXAMPLE OF PRODUCTION OF COMPOUND (1b)

The compound (3A-2) obtained in the same manner as in Example 15 is charged into a flask, and at the outlet of the flask, a system constituted by the connection of a trap kept to be -78°C and a vacuum pump in order is equipped. When the pressure of the system is reduced by a vacuum pump and then the flask is heated, liquid is distilled into a trap, and such a distillate contains impurities. Purification is carried out by distillation under reduced pressure to obtain the following compound (1b).

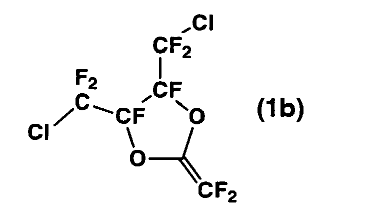

### EXAMPLE 17

### EXAMPLE OF HOMOPOLYMERIZATION OF COMPOUND (1b)

The compound (1b) obtained in the same manner as in Example 16 and ((CH₃)₂CHOCOO)₂ are put into a glass ampoule, and freeze-deaerated and sealed. The ampoule is heated in an oven at 50°C for 20 hours to obtain a solid material. The solid material is dissolved in R-225 and then purified by reprecipitation using hexane. Further, washing is repeated twice with hexane and then vacuum-dried at 50°C for 16 hours to obtain a polymer having the following monomer units (2b). Then, the polymer is dissolved in CCl₂FCF₂CClFCClF₂ to obtain 10 mass% of a solution composition. Using a PTFE sheet as a substrate, the solution composition is applied on a substrate by casting, and then dried at 150°C for 1 hour to form a coating film on the surface of the substrate.

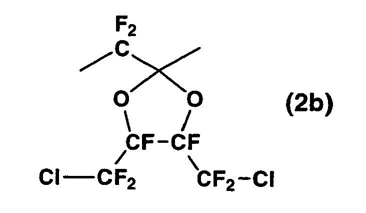

### INDUSTRIAL APPLICATION

The compound provided by the present invention is a compound having a perfluoro(2-methylene-1,3-dioxolane) structure and having a perfluoro(partially chlorinated monovalent saturated hydrocarbon) group at the 4-position and/or 5-position of the structure, and is a novel compound which is useful as a polymerizable monomer. The fluoropolymer comprising monomer units derived from the compound, is characterized by having excellent mechanical strength and transparency, high glass transition point, and high refractive index.

The present invention provides a novel compound having a structure characterized by having a perfluoro(2-methylene-1,3-dioxolane) skeleton having a perfluoro(partially chlorinated monovalent saturated hydrocarbon) group at the 4-position and/or 5-position. A fluoropolymer obtainable by polymerizing the compound is useful as an optical material since such a polymer is excellent in mechanical strength or transparency and has high glass transition point and a high refractive index. For example, it is useful for an optical waveguide material or a 45° mirror material for a 90° optical path conversion of an optical/electrical circuit board.

## Claims

1. A compound represented by the following formula (1): 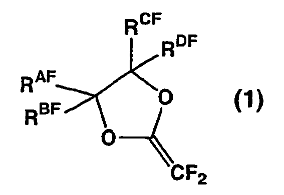 R^{AF}, R^{BF}, R^{CF}, R^{DF}: each independently is a fluorine atom, a chlorine atom, a perfluoro monovalent saturated hydrocarbon group or a perfluoro(partially chlorinated monovalent saturated hydrocarbon) group, provided that at least one of R^{AF}, R^{BF}, R^{CF} and R^{DF} is a perfluoro(partially chlorinated monovalent saturated hydrocarbon) group.

2. The compound according to Claim 1, wherein each of R^{AF} and R^{CF} which are independent of each other, is a perfluoro(partially chlorinated monovalent saturated hydrocarbon) group, and R^{BF} and R^{DF} are fluorine atoms, or R^{AF} is a perfluoro(partially chlorinated monovalent saturated hydrocarbon) group, and R^{BF}, R^{CF} and R^{DF} are fluorine atoms.

3. The compound according to Claim 1 or 2, wherein the perfluoro(partially chlorinated monovalent saturated hydrocarbon) group is a C₁₋₆ perfluoro(partially chlorinated alkyl) group.

4. The compound according to any one of Claims 1 to 3, wherein the ratio of the number of chlorine atoms in the compound represented by the formula (1) to the number of carbon atoms in the same compound, is from 0.1 to 0.5.

5. A fluoropolymer comprising monomer units represented by the following formula (2): 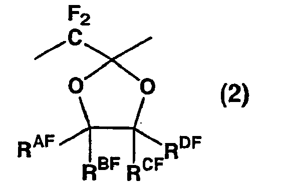 R^{AF}, R^{BF}, R^{CF}, R^{DF}: each independently is a fluorine atom, a chlorine atom, a perfluoro monovalent saturated hydrocarbon group or a perfluoro(partially chlorinated monovalent saturated hydrocarbon) group, provided that at least one of R^{AF}, R^{BF}, R^{CF} and R^{DF} is a perfluoro(partially chlorinated monovalent saturated hydrocarbon) group.

6. The fluoropolymer according to Claim 5, wherein each of R^{AF} and R^{CF} which are independent of each other, is a perfluoro(partially chlorinated monovalent saturated hydrocarbon) group, and R^{BF} and R^{DF} are fluorine atoms, or R^{AF} is a perfluoro(partially chlorinated saturated monovalent hydrocarbon) group, and R^{BF}, R^{CF} and R^{DF} are fluorine atoms.

7. The fluoropolymer according to Claim 5 or 6, wherein the perfluoro(partially chlorinated monovalent saturated hydrocarbon) group is a C₁₋₆ perfluoro (partially chlorinated alkyl) group.

8. The fluoropolymer according to any one of Claims 5 to 7, wherein the ratio of the number of chlorine atoms in the monomer units represented by the formula (2) to the number of carbon atoms in the same monomer units, is from 0.1 to 0.5.

9. The fluoropolymer according to any one of Claims 5 to 8, which comprises at least one type of monomer units represented by the formula (2).

10. The fluoropolymer according to any one of Claims 5 to 9, which has a number average molecular weight of from 5,000 to 5,000,000.

11. A process for producing a fluoropolymer comprising monomer units represented by the following formula (2), **characterized by** polymerizing a compound represented by the following formula (1), or copolymerizing a compound represented by the following formula (1) with another monomer polymerizable with the compound: 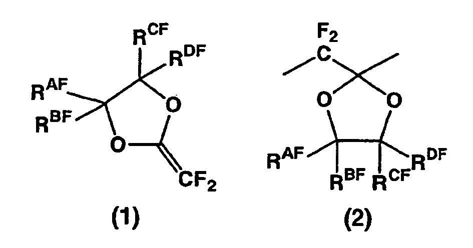 R^{AF}, R^{BF}, R^{CF}, R^{DF}: each independently is a fluorine atom, a chlorine atom, a perfluoro monovalent saturated hydrocarbon group or a perfluoro(partially chlorinated monovalent saturated hydrocarbon) group, provided that at least one of R^{AF}, R^{BF}, R^{CF} and R^{DF} is a perfluoro(partially chlorinated monovalent saturated hydrocarbon) group.

12. An optical material comprising as an effective component the fluoropolymer as defined in any one of Claims 5 to 10.
